# EUROPEAN PATENT APPLICATION

(11) **EP 4 011 407 A1**
(43) Date of publication of application: **15.06.2022**
(21) Application number: 20850095.9
(22) Date of filing: 01.07.2020
(51) Int. Cl.: A61L 9/14, A61L 9/20

(54) **GAS PROCESSING DEVICE, GAS PROCESSING SYSTEM, AND GAS PROCESSING METHOD**

(30) Priority: 08.08.2019 JP 2019146324
(71) Applicant: Ushio Denki Kabushiki Kaisha, Tokyo 100-8150 (JP)
(72) Inventor: NAITO,Keisuke, Tokyo 100-8150 (JP)
(74) Representative: Tomerius, Isabel
(86) International application number: PCT/JP2020/025880
(87) International publication number: WO 2021/024653

(57) **Abstract**

Provided is a gas treatment apparatus whereby VOC-containing gases to be treated can be processed efficiently with the use of light having a wavelength of 200 nm or less. A gas treatment apparatus includes: a light source radiating light having a wavelength of 200 nm or less; a housing having an inlet port and an outlet port for a gas to be treated that contains a VOC and oxygen, and forming a gas treatment space between the inlet port and the outlet port, in which the gas to be treated is guided to a region within 15 mm of a light-emitting surface of the light source; and a water supply mechanism supplying water in mist form between the gas treatment space and the outlet port.

## Description

### TECHNICAL FIELD

This invention relates to a gas treatment apparatus, a gas treatment system, and a gas treatment method, and more particularly to a device and method for processing VOC (Volatile Organic Compounds)-containing gases to be treated.

### BACKGROUND ART

Gas treatment apparatuses that decompose gases containing chemical pollutants such as malodorous components by ultraviolet have been developed in recent years. It has hitherto been known, in such a gas treatment apparatus, to supply moisture for enhancing the gas treatment efficiency. For example, Patent Document 1 listed below describes an apparatus in which a kitchen waste gas is passed through a cleaning container sprayed with a cleaner solution containing ozone gas and titanium dioxide and irradiated with UV to remove the odor. Patent Document 2 listed below describes efficient decomposition and removal of chemical pollutants by ultraviolet irradiation, in which cleaning water is supplied to a UV irradiation space to facilitate generation of hydroxyl radicals.

### PRIOR ART DOCUMENTS

### PATENT DOCUMENT

Patent Document 1: JP-A-2002-143637
Patent Document 2: JP-A-2006-204683

### SUMMARY OF THE INVENTION

### PROBLEMS TO BE SOLVED BY THE INVENTION

The present inventor is considering a use of light having a wavelength of 200 nm or less, which belongs to the wavelength range of vacuum ultraviolet and which is different from that of the Patent Documents mentioned above, in order to decompose gases to be treated which contain VOCs that are chemical pollutants.

There are currently no findings regarding efficient treatment of VOC-containing gases to be treated with the use of light having a main wavelength of 200 nm or less.

An object of the present invention is to provide a gas treatment apparatus, a gas treatment system, and a gas treatment method whereby VOC-containing gases to be treated can be processed efficiently with the use of light having a wavelength of 200 nm or less.

### MEANS FOR SOLVING THE PROBLEMS

A gas treatment apparatus according to the present invention includes:
a light source radiating light having a wavelength of 200 nm or less;
a housing having an inlet port and an outlet port for a gas to be treated that contains a VOC and oxygen, and forming a gas treatment space between the inlet port and the outlet port, in which the gas to be treated is guided to a region within 15 mm of a light-emitting surface of the light source; and
a water supply mechanism supplying water in mist form between the gas treatment space and the outlet port.

VOCs herein generally stand for volatile organic compounds in gaseous form at ordinary temperature and normal pressure, which are substances that cause indoor and outdoor air pollution. As will be described later in more detail, a gas to be treated containing VOCs and oxygen (e.g., air with VOCs mixed therein) is irradiated with light having a wavelength of 200 nm or less, whereby some substances belonging to VOCs transform into hydrophilic particles which then efficiently adsorb water in mist form and drop down in the gas treatment apparatus. The light having a wavelength of 200 nm or less preferably has a main peak wavelength of 160 to 180 nm.

The housing may further include a reservoir tank that stores water, and the reservoir tank may be provided on a side closer to the outlet port relative to the gas treatment space. The housing may have the light source, the water supply mechanism, and the reservoir tank arranged inside successively from the top vertically downwards. This increases the chance of the gas to be treated making contact with the liquid surface of the reservoir tank for effective trapping of VOCs in the reservoir tank.

The gas treatment apparatus may further include a discharge mechanism that discharges water that has accumulated in the reservoir tank, and the outlet port may be located higher than the reservoir tank.

The gas treatment apparatus may further include a cleaning mechanism that ejects a cleaning liquid toward the light-emitting surface. This allows the light source to recover its irradiation intensity to enhance the VOC treatment capability.

A gas treatment system according to the present invention may include a plurality of the gas treatment apparatuses described above,
the plurality of gas treatment apparatuses being disposed such that the outlet port of one gas treatment apparatus is connected to the inlet port of another gas treatment apparatus via an intermediate flow path,
the intermediate flow path having a flow path length of 50 cm or more, or a mist removal filter.

This way, the gas to be treated can be processed via multiple stages using a plurality of gas treatment apparatuses, so that the VOC concentration can be further reduced.

The gas treatment system may further include a controller controlling the plurality of gas treatment apparatuses,
the controller executing control such that a light source of at least one of the gas treatment apparatuses is turned on to perform gas treatment while a light source of another gas treatment apparatus is turned off. This allows for gas treatment in another gas treatment apparatus during a maintenance check of one gas treatment apparatus, so that the treatment can continue without stopping the inflow of VOCs.

A gas treatment method of the present invention includes: an introducing step (a) of guiding a gas to be treated that contains a VOC and oxygen to a region within 15 mm of a light-emitting surface of a light source that radiates light having a wavelength of 200 nm or less;
an irradiation step (b) of irradiating the gas to be treated that has been guided to the region with the light; and
a water supply step (c) of supplying water in mist form to the gas to be treated after the irradiation step.

In the gas treatment method described above, the irradiation step (b) and the water supply step (c) may be performed in at least one gas treatment apparatus in a gas treatment system having a plurality of gas treatment apparatuses, and a cleaning step (d) in which the light-emitting surface is cleaned, with the light source being turned off, may be performed in another gas treatment apparatus.

### EFFECT OF THE INVENTION

A VOC-containing gas to be treated can thus be processed efficiently.

### BRIEF DESCRIPTION OF THE DRAWINGS

Fig. 1 is a diagram illustrating a gas treatment apparatus according to a first embodiment.
Fig. 2 is a diagram illustrating a gas treatment apparatus according to a first variation example of the first embodiment.
Fig. 3A is a diagram illustrating a gas treatment apparatus according to a second variation example of the first embodiment.
Fig. 3B is a cross-sectional view of line A-A in Fig. 3A.
Fig. 4 is a diagram illustrating a gas treatment apparatus according to a second embodiment.
Fig. 5 is a diagram illustrating a gas treatment apparatus according to a variation example of the second embodiment.
Fig. 6 is a diagram illustrating a gas treatment system according to a third embodiment.
Fig. 7 is a diagram illustrating a gas treatment system according to a variation example of the third embodiment.

### MODE FOR CARRYING OUT THE INVENTION

The gas treatment apparatus according to the present invention will be described with reference to the drawings. It should be noted that the drawings disclosed herein merely show schematic illustrations. Namely, the dimensional ratios on the drawings do not necessarily reflect the actual dimensional ratios, and the dimensional ratios are not necessarily the same between the drawings.

### < First embodiment>

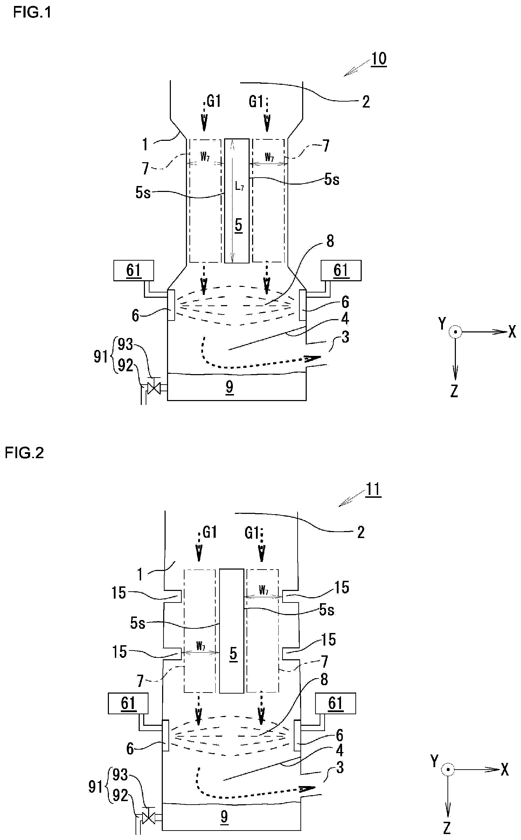

Fig. 1 illustrates a gas treatment apparatus according to the first embodiment of the present invention. In the following, a description will be given with reference to an XYZ coordinate system as required, in which a horizontal plane is referred to as XY plane and a vertical direction is referred to as Z direction. In describing directions herein, when distinguishing whether the direction is positive or negative, the positive or negative symbol will be added, such as "+X direction" or "-X direction". Directions will simply be described as "X direction" when there is no distinction between positive or negative. Namely, where described simply as "X direction" herein, both "+X direction" and "-X direction" are included. The same applies to Y direction and Z direction.

The gas treatment apparatus 10 includes a housing 1 and a light source accommodated inside the housing 1. An excimer lamp 5 formed by a tubular body extending in one direction (Z direction in the drawing) is illustrated as one example of the light source. The gas treatment apparatus 10 is a device having a function of decomposing VOCs by irradiating a VOC-containing gas (hereinafter referred to as "gas to be treated") G1 with light of a predetermined wavelength (ultraviolet) from a light source (which corresponds to the excimer lamp 5 in Fig. 1).

VOCs stand for volatile organic compounds in gaseous form at ordinary temperature and normal pressure, which are substances that cause indoor and outdoor air pollution. Typical substances that contain VOCs are toluene, formaldehyde, xylene, benzene, methanol, isopropyl alcohol, acetone, methyl isobutyl ketone, ethyl acetate, and so on. The gas to be treated G1 flows generally in directions indicated with broken line arrows inside the housing 1.

The gas to be treated G1 is introduced from an introducing section for gas to be treated (not shown). The introducing section for gas to be treated corresponds for example to an outlet port of a chamber space such as a draft chamber or the like. A blower (not shown) is disposed downstream of the gas treatment apparatus 10. By driving the blower, the gas to be treated G1 introduced from the introducing section for gas to be treated is sucked and flows into the gas treatment apparatus 10 from an inlet port 2. The gas to be treated G1 flows through the gas treatment space 7 and is discharged from an outlet port 3. The discharged treated gas G1 is sent to a downstream treated gas collection tank (not shown) or an air release port (not shown).

The draft chamber and other spaces are often placed in an atmospheric environment. Therefore, generally, the gas to be treated G1 contains not just VOCs but also oxygen and water vapor in the atmosphere. Oxygen and water vapor are sources for producing hydroxyl radicals that are necessary for decomposition of the gas to be treated G1. In the case of decomposing or removing VOCs that were created in a special environment without the air or with little air, oxygen and water vapor may be added to VOCs intentionally to generate the gas to be treated G1.

### [Gas treatment space]

The gas to be treated G1 passing through the gas treatment space 7 (inside the one-dot chain line frames in Fig. 1) is irradiated with light from the excimer lamp 5. The excimer lamp 5 radiates light having a wavelength of 200 nm or less. As will be described later in more detail, hydroxyl radicals are generated from the gas to be treated G1 by the light having a wavelength of 200 nm or less. It is particularly preferable to radiate light having a main peak wavelength of 160 to 180 nm. The light having a main peak wavelength of 160 to 180 nm is part of a wavelength range that is called VUV (Vacuum Ultra Violet). The light has a shorter wavelength than that of ultraviolet emitted in hitherto known low-pressure mercury-vapor lamps (e.g., light having a main peak wavelength of 185 nm). A typical example of light sources that generate light having a main peak wavelength of 160 to 180 nm is the excimer lamp hermetically filled with gases containing xenon (Xe), a typical peak wavelength of the excimer lamp generally being 172 nm. In the following description, an expression "172 nm light" is sometimes used instead of "light having a main peak wavelength of 160 to 180 nm". There is no particular difference in characteristics between "172 nm light" and "light having a main peak wavelength of 160 to 180 nm" in regard to the contents herein. Since any specification relating to "172 nm light" hereinafter can also apply to "light having a main peak wavelength of 160 to 180 nm", the "172 nm light" mentioned hereinafter can be read as "light having a main peak wavelength of 160 to 180 nm".

The excimer lamp 5 used in this embodiment has a length L₇ in the tube axial direction (Z direction in the drawing) of 540 mm and a tube diameter of 20 mm. While the excimer lamp 5 in this embodiment is a single type, a plurality of lamps arranged side by side may also be used. There are no limitations on the shape, number, or direction of installation of the lamp.

The gas treatment space 7 has a size that accommodates a clearance distance W₇ that is a length in X direction in the drawing from the light-emitting surface 5s of the excimer lamp 5 to an inner wall of the housing 1. The clearance distance W₇ is 15 mm or less. This means that the gas to be treated G1 is guided to a region within 15 mm of the light-emitting surface 5s of the excimer lamp 5. The reason why the gas to be treated G1 is guided to the region within 15 mm of the light-emitting surface 5s of the excimer lamp 5 is as follows.

At first it was assumed that the 172 nm light would not reach regions over 6 mm from the light-emitting surface 5s because the 172 nm light would be absorbed by oxygen molecules and the light energy would disappear when the distance from the light-emitting surface 5s exceeded 6 mm.

However, as a result of intensive research, even if the clearance distance W₇ was over 6 mm, VOCs passing through regions away from the light-emitting surface 5s could be relatively prevented from traveling through the gas treatment space 7 without being decomposed if the clearance distance W₇ was 15 mm or less. The reason for this is that the flow of the gas to be treated G1 in the gas treatment space 7 is turbulent, constantly changing the distance from the light-emitting surface 5s rather than laminar, i.e., in layers parallel (in Z direction in the drawing) to the excimer lamp 5. Namely, if the gas treatment space 7 has a clearance distance W₇ of 15 mm or less, it is often the case that the gas to be treated G1 present in regions of more than the clearance distance W₇ of 6 mm in the gas treatment space 7 enters the region within the clearance distance W₇ of 6 mm where the light reaches, somewhere in the gas treatment space 7, because of the turbulent flow. With a large clearance distance W₇, the flow rate of the gas to be treated G1 passing through the gas treatment space 7 can be reduced, which consequently allows a sufficient time for the optical reaction of the gas to be treated G1.

It is particularly preferable to set the clearance distance W₇ within 10 mm. That way, VOCs traveling through a region away from the light-emitting surface 5s can be prevented from passing through the gas treatment space 7 without being decomposed.

The clearance distance W₇ need not necessarily be the inner diameter of the tubular housing 1 that has a uniform inner diameter along the air flow direction. Fig. 2 illustrates a gas treatment apparatus according to a first variation example of the first embodiment. The housing 1 of the gas treatment apparatus 11 has protruded parts 15 projecting radially toward the center where the excimer lamp 5 is, i.e., the inner diameter of the housing 1 varies depending on the position in the Z direction. The protruded parts 15 cause the flow of the gas to be treated G1 to be concentrated near the light-emitting surface 5s. When there is a protruded part 15, the clearance distance W₇ is a distance from the light-emitting surface 5s of the lamp to a distal end of the protruded part 15. In the case where such protruded parts 15 are provided, the clearance distance W₇ from the light-emitting surface 5s of the excimer lamp 5 to the housing 1 in other parts than the protruded parts 15 may be set such as not to allow the light to reach the gas to be treated G1 at least partly (e.g., more than 15 mm).

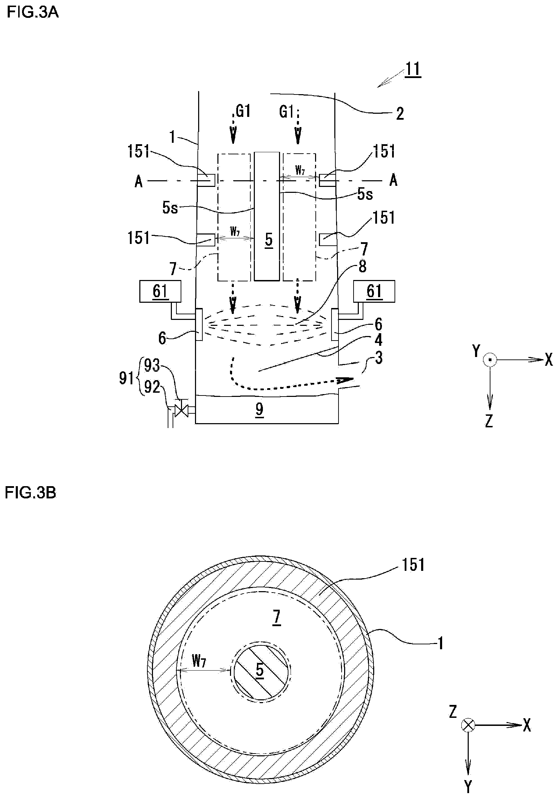

Fig. 3A illustrates a gas treatment apparatus according to a second variation example of the first embodiment. The tubular housing 1 includes baffle plates 151 protruding from the inner wall toward the excimer lamp 5. The baffle plates 151, similarly to the protruded parts 15, cause the flow of the gas to be treated G1 to be concentrated near the light-emitting surface 5s. Fig. 3B is a cross-sectional view of line A-A in Fig. 3A. In this variation example, the baffle plate 151 is annular and in contact with the inner walls of the housing 1. The baffle plate 151 is not limited to the shape of this variation example and may adopt various shapes.

Irradiating the gas to be treated G1 with the 172 nm light produces hydroxyl radicals from the water vapor and oxygen contained in the gas to be treated G1. The large amount of generated hydroxyl radicals decompose the VOC gas and transform the VOC gas into carbon dioxide and water.

However, when the gas contains some VOCs that are hard to decompose such as toluene, the gas would sometimes pass through the gas treatment space 7 without the VOCs being completely decomposed by the chemical reaction with hydroxyl radicals. Some gases containing VOCs such as toluene are known to be hardly soluble to water, and it has been considered difficult to remove such VOCs by dissolving them in water.

As a result of intensive research, the present inventor discovered that when a gas containing a VOC that exhibits low solubility to water such as toluene was irradiated with the 172 nm light, while the VOC was not completely decomposed, the VOC showed hydrophilicity due to hydroxyl groups imparted to some of the side chains, and the VOC coagulated into particles in the gas. It was also found out that when water in mist form was supplied to these hydrophilic VOC particles, the VOC particles adsorbed water and increased in mass, which led to the present invention.

### [Mist nozzle]

Mist nozzles 6, which are a water supply mechanism that supplies water in mist form, are disposed in a water supply space between the gas treatment space 7 and the outlet port 3. The mist nozzle 6 is different from a shower nozzle that simply sprinkles liquid, but supplies water in mist form by spraying liquid. Mist form refers to an aerosol of liquid particles suspended in the air. The liquid sprayed from the mist nozzle 6 is supplied from a liquid supply unit 61. The liquid contains a large amount of water molecules, and may contain other components than water molecules. There may be a single liquid supply unit 61 connected to the nozzles via a branch, or a plurality of units each connected to each nozzle. In this embodiment, the mist nozzles 6 are provided in inner walls of the housing 1 and spray a liquid radially toward the center of the housing 1.

Hydrophilic VOC particles that have exited the gas treatment space 7 make contact with liquid particles 8 sprayed from the mist nozzles 6, adsorb the liquid particles 8, and increase in mass. The VOC particles with increased mass drop out from the gas flow toward the outlet port 3 by their own weight down into the reservoir tank 9 to be described later. Thus, the amount of VOCs that flow out from the outlet port 3 can be reduced.

When the liquid particles 8 have a small size, or in a gaseous form (water vapor), there is less effect of increasing the mass of the VOC particles when the liquid is adsorbed, in which case the VOC particles will not drop out from the gas flow toward the outlet port 3 and flow out from the outlet port 3 with the gas flow. Accordingly, the liquid particles 8 supplied from the mist nozzles 6 should preferably have a particle diameter of 1 µm or more, and more preferably 10 µm or more. This way, the mass of the VOC particles can be effectively increased so that the VOC particles can readily drop out and down from the gas flow toward the outlet port 3.

Moreover, the liquid particles 8 supplied from the mist nozzles 6 should preferably have a particle diameter of 4000 µm or less, and more preferably 1000 µm or less. The smaller the particle diameter of the liquid particles 8, the larger the number of liquid particles 8 occupying the space, so that the overall total surface area of the liquid particles 8 can be increased. An increased overall total surface area of the liquid particles 8 increases the contact area with VOC particles, so that adsorption of liquid particles 8 on the VOC particles can be effectively enhanced. The particle diameter of the liquid particles 8 is measured by a laser diffraction method.

In this embodiment, the excimer lamp 5 is disposed above the mist nozzles 6 (opposite direction from the vertical direction, or Z direction in the drawing). The reason for this is as follows. Sometimes the excimer lamp 5 has electrodes disposed on the light-emitting surface 5s for supplying power to the excimer lamp 5. Water depositing on these electrodes could damage the excimer lamp 5 due to a concentrated discharge caused by a local change in resistance between the electrodes. If, however, the excimer lamp 5 is disposed above the mist nozzles 6, the liquid particles 8 supplied from the mist nozzles 6 will flow down (vertical direction) along the gravity direction and the gas flow, and the liquid particles 8 will hardly deposit on the light-emitting surface 5s of the excimer lamp 5 so that the excimer lamp 5 is less susceptible to the risk of damage.

### [Reservoir tank]

In this embodiment, the reservoir tank 9 is disposed at the bottom of the housing 1 so that VOC particles dropping down from the gas flow toward the outlet port 3 can be accumulated. The bottom referred to here corresponds to a part of the housing located at an end in the downward vertical direction (+Z direction). The reservoir tank 9 storing the liquid provides the effect of trapping VOCs by adsorption, through contact with the hydrophilic VOC gas. Thus the amount of VOCs that flow out from the outlet port 3 can be reduced. Therefore, the gas treatment apparatus 10 of this embodiment has the light source (excimer lamp 5), the mist nozzles 6, and the reservoir tank 9 successively from the top (Z direction in the drawing) downwards in the vertical direction (Z direction in the drawing). As long as the reservoir tank 9 is located on the side closer to the outlet port 3 relative to the gas treatment space 7, the reservoir tank 9 need not necessarily be disposed vertically below the gas treatment space 7 or the excimer lamp 5.

A flow control plate 4 that changes the flow of the gas to be treated G1 may be provided between the outlet port 3 and the mist nozzles 6 as in this embodiment. The flow control plate 4 guides the gas to be treated G1 that has passed the mist nozzles 6 to make contact with the liquid surface of the reservoir tank 9 before it is discharged from the outlet port 3. This increases the chance of VOCs making contact with the liquid surface of the reservoir tank 9 for effective trapping of VOCs in the reservoir tank 9.

Preferably, a discharge mechanism 91 may be provided for discharging the water accumulated in the reservoir tank 9 from the housing 1 of the gas treatment apparatus. The discharge mechanism 91 may include, for example, a liquid drain pipe 92 connected to the reservoir tank 9, and an open/close valve 93 that opens or closes the liquid drain pipe 92. The open/close valve 93 may be opened upon detection of a certain level of liquid amount being exceeded. Alternatively, the open/close valve 93 may be opened at constant time intervals. The outlet port 3 should preferably be positioned higher than the reservoir tank 9 (opposite direction from the vertical direction). This way, the liquid that has accumulated in the reservoir tank 9 can be prevented from flowing out of the outlet port 3.

### <Second embodiment>

The second embodiment is similar in configuration to the first embodiment except for the following points. Therefore, description of the common points will be omitted. The same applies to the third embodiment.

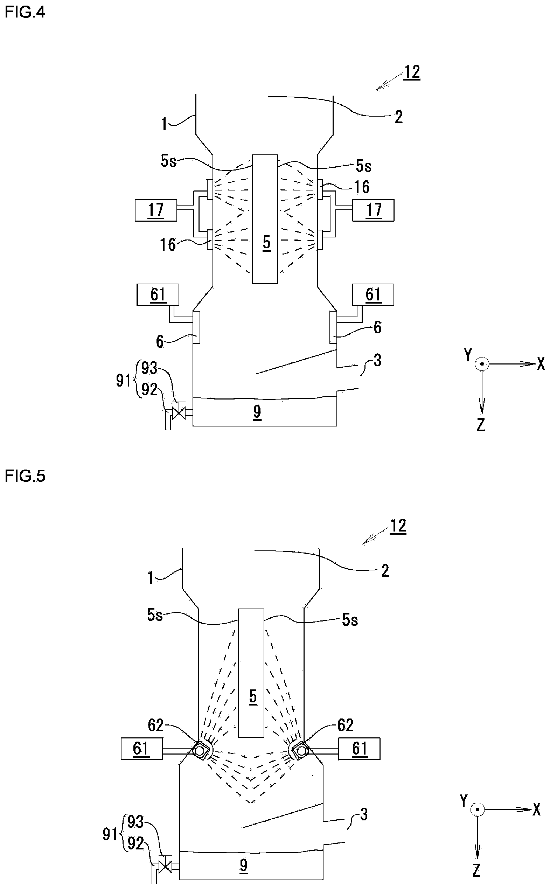

Fig. 4 illustrates a gas treatment apparatus according to the second embodiment. The gas treatment apparatus 12 includes cleaning nozzles 16, which are a cleaning mechanism that ejects a cleaning liquid toward the light-emitting surface 5s of the excimer lamp 5. The cleaning nozzles 16 are used while the excimer lamp 5 is turned off. Sometimes, particles originating from VOCs produced by irradiation adhere on the light-emitting surface 5s of the excimer lamp 5 as foreign substances, obscuring the light-emitting surface 5s and reducing the irradiation intensity. It has been revealed that a supply of a cleaning liquid on the light-emitting surface 5s could dissolve the adhering particles and remove the obscuration on the light-emitting surface 5s. Therefore, ejecting a cleaning liquid toward the light-emitting surface 5s of the excimer lamp 5 to wash off the foreign substances adhering on the light-emitting surface 5s could boost the irradiation intensity of the excimer lamp 5. The cleaning liquid after the washing accumulates in the reservoir tank 9. The liquid that has accumulated in the reservoir tank 9 is discharged as required using the discharge mechanism 91. These operations of the gas treatment apparatus are controlled by a controller (not shown in Fig. 4) of the gas treatment apparatus 12.

The cleaning liquid is supplied from a cleaning liquid supply unit 17. There may be a single cleaning liquid supply unit 17, and this single cleaning liquid supply unit 17 may be connected to each of the cleaning nozzles 16 via a branch. Alternatively, there may be a plurality of cleaning liquid supply units 17, and each of the cleaning liquid supply units 17 may be connected to each of the cleaning nozzles 16. Particles adhering on the light-emitting surface are often soluble to water, and therefore the cleaning liquid should preferably contain a large amount of water. Water may be used, for example, as the cleaning liquid, or water containing a cleaning liquid component may be used. If the cleaning liquid component is the same as that supplied from the mist nozzles 6, the cleaning liquid supply unit 17 may be integrated with the liquid supply unit 61 that supplies liquid to the mist nozzles 6. After the cleaning, the excimer lamp 5 is turned on after the light-emitting surface 5s has been dried by blown air or the like. The cleaning may be performed at constant time intervals, or may be performed based on measurement results of irradiation intensity.

Fig. 5 illustrates a gas treatment apparatus according to a variation example of the second embodiment. Nozzles 62 are configured to be rotatable so as to be able to supply both of the water to be adsorbed on VOC particles during the gas treatment and the cleaning water for the excimer lamp 5. The nozzles 62 are directed horizontally or toward the downstream direction to function as a water supply mechanism that supplies water to be adsorbed on VOC particles during the gas treatment. When cleaning the excimer lamp 5, the nozzles are rotated toward the light-emitting surface 5s to function as a cleaning mechanism that ejects cleaning water. The nozzles 62 may be designed to be movable in the up and down direction.

### <Third embodiment>

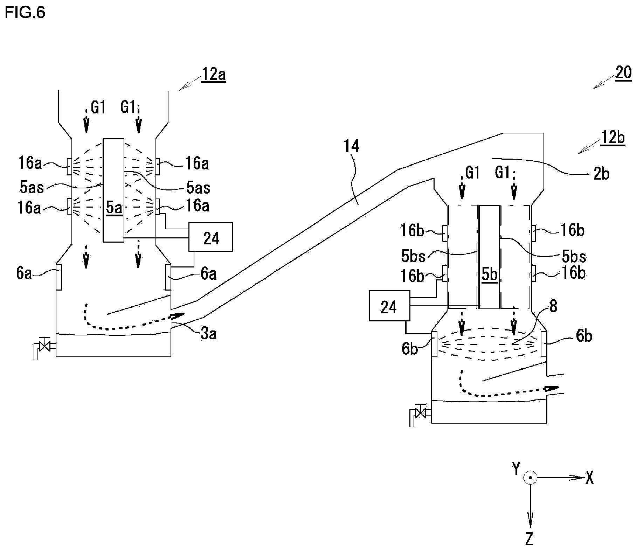

Fig. 6 illustrates a gas treatment system according to the third embodiment, which includes a plurality of gas treatment apparatuses 12 of the second embodiment. In this embodiment, the gas treatment system 20 includes a gas treatment apparatus 12a close to the introducing section for gas to be treated, and a gas treatment apparatus 12b disposed on the side of the outlet port 3a of the gas treatment apparatus 12a, and a controller 24. The gas treatment system 20 has the gas treatment apparatuses disposed in series such that the outlet port 3a of the gas treatment apparatus 12a is connected to the inlet port 2b of the gas treatment apparatus 12b via an intermediate flow path 14. The controller 24 is electrically connected to the excimer lamps (5a, 5b), mist nozzles (6a, 6b), and cleaning nozzles (16a, 16b).

Fig. 6 shows the gas treatment system 20 in which the excimer lamp 5a in the gas treatment apparatus 12a is turned off and the light-emitting surface 5as is cleaned, while the excimer lamp 5b in the gas treatment apparatus 12b is turned on and the liquid particles 8 are supplied from the mist nozzles 6b. Since the excimer lamp 5a is turned off, the light-emitting surface 5as of the excimer lamp 5a can be cleaned without damaging the excimer lamp 5a. The controller 24 executes control such that, when the cleaning in the gas treatment apparatus 12a is finished, the excimer lamp 5b in the gas treatment apparatus 12b is turned off and the light-emitting surface 5bs is cleaned, while the excimer lamp 5a in the gas treatment apparatus 12a is turned on and liquid particles 8 are supplied from the mist nozzles 6a. Thus, while gas treatment is performed in one gas treatment apparatus, maintenance work of the other gas treatment apparatus is possible. Maintenance work includes not only the cleaning of the light-emitting surface 5s of the excimer lamp 5, but also replacement of excimer lamps 5, inspection of gas treatment apparatuses, and so on.

The intermediate flow path 14 may include a flow path length of 50 cm or more, or a mist removal filter. When the excimer lamp 5a is turned off and the light-emitting surface 5as is cleaned in the gas treatment apparatus 12a, the intermediate flow path 14 having a flow path length of 50 cm or more causes the mist component in the cleaning liquid to drop down the intermediate flow path 14 and prevents it from flowing into the gas treatment apparatus 12b. This consequently prevents water from adhering on the electrodes of the excimer lamp 5b that is being lit in the gas treatment apparatus 12b, thereby to prevent damage to the excimer lamp 5b.

To stop the mist component from flowing into the downstream gas treatment apparatus 12b, a mist removal filter may be provided in the intermediate flow path 14. The mist removal filter may be made of a humidity adjusting material, zeolite, diatomite and the like, and provided such as to block the intermediate flow path 14, or formed on inner walls of the intermediate flow path 14. The intermediate flow path 14 may be formed linearly, or in a winding manner.

If the inlet port 2b of the gas treatment apparatus 12b is located higher than the outlet port 3a of the gas treatment apparatuses 12a, the mist component dropping down the intermediate flow path 14 is prevented from entering the gas treatment apparatus 12b and can be collected in the gas treatment apparatus 12a.

When no maintenance work such as cleaning is performed in both gas treatment apparatuses, the excimer lamps (5a, 5b) of both gas treatment apparatuses may be turned on. This way, the VOC concentration of the gas to be treated G1 can be further reduced.

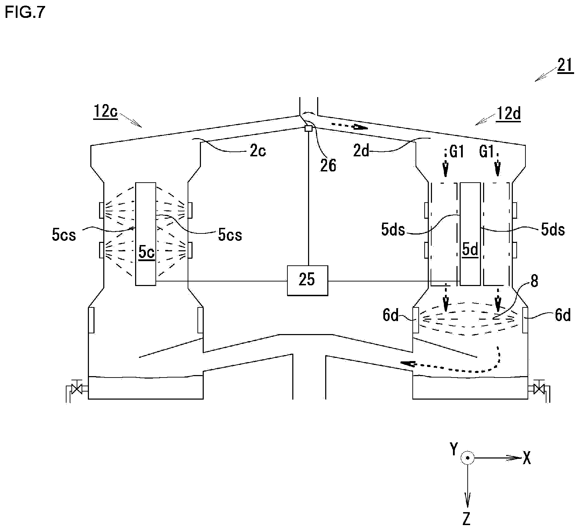

Fig. 7 illustrates a gas treatment system according to a variation example of the third embodiment. In the gas treatment system 21, a gas treatment apparatus 12c and a gas treatment apparatus 12d are disposed in parallel and not in series. A damper 26 is provided in passages that lead to the respective inlet ports 2c and 2d of the devices. Using the damper 26, the passage leading to one gas treatment apparatus (in Fig. 7, gas treatment apparatus 12c) is closed, and the passage leading to the other gas treatment apparatus (in Fig. 7, gas treatment apparatus 12d) is opened.

This allows the gas to be treated G1 to be processed in the other gas treatment apparatus (in Fig. 7, gas treatment apparatus 12d), i.e., allows the gas to be treated G1 to be irradiated with light from the light-emitting surface 5ds of the excimer lamp 5d, and liquid particles 8 to be supplied from the mist nozzles 6d. Meanwhile, maintenance work (such as cleaning of the light-emitting surface 5cs of the excimer lamp 5c) can be performed in one gas treatment apparatus (in Fig. 7, gas treatment apparatus 12c).

A driver of the damper 26 is electrically connected to a controller 25. The controller 25 may switch the orientation of the damper 26 atconstanttime intervals. Alternatively, the irradiation intensity in the gas treatment apparatus with the lamp on may be monitored with an illuminance sensor, and the orientation of the damper 26 may be switched when there is a drop in the irradiation intensity. A 3-way valve, or shutters or the like provided in respective flow paths can also act as the damper 26.

While the gas treatment systems (20, 21) in the third embodiment are configured with two gas treatment apparatuses, the system may be configured with three or more gas treatment apparatuses.

A gas treatment method of the present invention includes: an introducing step (a) of guiding a gas to be treated G1 to a gas treatment space 7 within 10 mm of the light-emitting surface 5s of the excimer lamp 5, an irradiation step (b) of irradiating the gas to be treated G1 that has been guided to the gas treatment space 7 with the light from the excimer lamp 5; and a water supply step (c) of supplying water in mist form to the gas to be treated G1 after the irradiation step.

Further, in the gas treatment method described above, the irradiation step (b) and the water supply step (c) may be performed in at least one gas treatment apparatus in a gas treatment system having a plurality of gas treatment apparatuses, and a cleaning step (d) in which the light-emitting surface 5s is cleaned, with the excimer lamp 5 being turned off, may be performed in another gas treatment apparatus.

The present invention is not limited to the embodiments described above in any way. Various improvements and modifications may be made without departing from the scope of the subject matter of the present invention.

### DESCRIPTION OF REFERENCE SIGNS

- 1: Housing
- 2, 2b, 2c, 2d: Inlet port
- 3, 3a: Outlet port
- 4: Flow control plate
- 5, 5a, 5b, 5c, 5d: Excimer lamp
- 5s, 5as, 5bs, 5cs, 5ds: Light-emitting surface
- 6, 6a, 6b, 6d: Mist nozzle
- 7: Gas treatment space
- 8: Liquid particle
- 9: Reservoir tank
- 10, 11, 12, 12a, 12b, 12c, 12d: Gas treatment apparatus
- 14: Intermediate flow path
- 15: Protruded part
- 16, 16a, 16b: Cleaning nozzle
- 17: Cleaning liquid supply unit
- 20, 21: Gas treatment system
- 24, 25: Controller
- 26: Damper
- 61: Liquid supply unit
- 62: Nozzle
- 91: Discharge mechanism
- 92: Liquid drain pipe
- 93: Open/close valve
- 151: Baffle plate

## Claims

1. A gas treatment apparatus comprising:
a light source radiating light having a wavelength of 200 nm or less;
a housing having an inlet port and an outlet port for a gas to be treated that contains a VOC and oxygen, and forming a gas treatment space between the inlet port and the outlet port, in which the gas to be treated is guided to a region within 15 mm of a light-emitting surface of the light source; and
a water supply mechanism supplying water in mist form between the gas treatment space and the outlet port.

2. The gas treatment apparatus according to claim 1, wherein the light source radiates light having a main peak wavelength of 160 to 180 nm.

3. The gas treatment apparatus according to claim 1 or 2, wherein the housing includes a reservoir tank that stores water,
the reservoir tank being provided on a side closer to the outlet port relative to the gas treatment space.

4. The gas treatment apparatus according to claim 3, wherein the housing has the light source, the water supply mechanism, and the reservoir tank arranged inside successively from the top.

5. The gas treatment apparatus according to claim 3 or 4, further comprising a discharge mechanism that discharges water that has accumulated in the reservoir tank,
the outlet port being located higher than the reservoir tank.

6. The gas treatment apparatus according to any one of claims 1 to 5, further comprising a cleaning mechanism that ejects a cleaning liquid toward the light-emitting surface.

7. A gas treatment system comprising: a plurality of the gas treatment apparatuses according to any one of claims 1 to 6,
the plurality of gas treatment apparatuses being disposed such that the outlet port of one gas treatment apparatus is connected to the inlet port of another gas treatment apparatus via an intermediate flow path,
the intermediate flow path having a flow path length of 50 cm or more, or a mist removal filter.

8. The gas treatment system according to claim 7, further comprising a controller controlling the plurality of gas treatment apparatuses,
the controller executing control such that a light source of at least one of the gas treatment apparatuses is turned on to perform gas treatment while a light source of another gas treatment apparatus is turned off.

9. A gas treatment method comprising: an introducing step (a) of guiding a gas to be treated that contains a VOC and oxygen to a region within 15 mm of a light-emitting surface of a light source that radiates light having a wavelength of 200 nm or less;
an irradiation step (b) of irradiating the gas to be treated that has been guided to the region with the light; and
a water supply step (c) of supplying water in mist form to the gas to be treated after the irradiation step.

10. The gas treatment method according to claim 9, wherein
the irradiation step (b) and the water supply step (c) are performed in at least one gas treatment apparatus in a gas treatment system having a plurality of gas treatment apparatuses, and
the gas treatment method further comprising a cleaning step (d) in which the light-emitting surface is cleaned, with the light source being turned off, is performed in another gas treatment apparatus.
